# EUROPEAN PATENT APPLICATION

(11) **EP 3 489 961 A1**
(43) Date of publication of application: **29.05.2019**
(21) Application number: 18202610.4
(22) Date of filing: 25.10.2018
(51) Int. Cl.: G16H 20/30, G16H 40/63, G16H 10/65, A61H 3/00, A63B 23/04

(54) **REHABILITATION SUPPORT APPARATUS, CONTROL METHOD, AND CONTROL PROGRAM**

(30) Priority: 24.11.2017 JP 2017226002
(71) Applicant: TOYOTA JIDOSHA KABUSHIKI KAISHA, Toyota-shi, Aichi-ken, 471-8571 (JP)
(72) Inventor: OTSUKI, Nobuhisa, Toyota-shi, Aichi-ken,, 471-8571 (JP); TANAKA, Nagisa, Toyota-shi, Aichi-ken,, 471-8571 (JP); TSUCHINAGA, Masayoshi, Toyota-shi, Aichi-ken,, 471-8571 (JP); KITO, Hodaka, Toyota-shi, Aichi-ken,, 471-8571 (JP)
(74) Representative: Winter, Brandl, Fürniss, Hübner, Röss, Kaiser, Polte - Partnerschaft mbB

(57) **Abstract**

A rehabilitation support apparatus according to a first exemplary aspect is configured to perform rehabilitation support by executing a training program for urging a trainee 900 to perform a predetermined motion and assisting the trainee 900 to perform the motion. The rehabilitation support apparatus includes a sensor configured to detect motion data from the motion performed by the trainee 900 and a control unit configured to execute the training program and generate training data of the trainee from the motion data detected by the sensor. Further, the control unit performs a writing process of writing the training data into an external memory according to a state of the training program.

## Description

### BACKGROUND

The present disclosure relates to a rehabilitation support apparatus, a control method and a control program.

People who have had their motor functions impaired make efforts to recover the motor functions by performing rehabilitation. Therefore, various apparatuses for appropriately performing such rehabilitation have been developed.

For example, a walking rehabilitation system, a part of which is attached to a user, and which assists his/her walking motions when he/she performs walking training has been known. For example, a walking rehabilitation system disclosed in Japanese Patent No. 6187208 includes: a treadmill; a floor reaction force sensor for measuring a reaction force acting on the treadmill; a leg robot attached to a lower limb of a user; and a distance measuring sensor for photographing the lower limb to which the leg robot is attached. The walking rehabilitation system further includes means for estimating a load (e.g., a pressure) on the sole of a user.

### SUMMARY

The rehabilitation system described above performs assisting operations according to various types of user's walking motions and generates data from various sensors in order to evaluate the user's walking motions. Further, a rehabilitation system may externally output the generated data. In such a case, it is desired that a rehabilitation system process information so that training motions of a user are smoothly performed.

The present disclosure has been made to solve the above-described problem and provide a rehabilitation support apparatus or the like capable of reducing an operation delay which interferes with training motions of a user.

A first exemplary aspect is a rehabilitation support apparatus configured to perform rehabilitation support by executing a training program for urging a trainee to perform a predetermined motion and assisting the trainee to perform the motion, the rehabilitation support apparatus including: a sensor configured to detect motion data from the motion performed by the trainee; and a control unit configured to execute the training program and generate training data of the trainee from the motion data detected by the sensor, in which the control unit performs a writing process of writing the training data into an external memory according to a state of the training program.

With such a structure, the rehabilitation support apparatus can control a timing of performing the wiring process. Thus, the rehabilitation support apparatus can perform the writing process so as not to interfere with training motions performed by a trainee.

Another exemplary aspect is a control method performed by a rehabilitation support apparatus configured to perform rehabilitation support by executing a training program for urging a trainee to perform a predetermined motion and assisting the trainee to perform the motion, the control method including: executing the training program for the trainee; detecting motion data from the motion performed by the trainee; generating training data of the trainee from the motion data; and performing a writing process of writing the training data into an external memory according to a state of the training program.

By performing the method described above, it is possible to perform the training data writing process so as not to interfere with training motions performed by a trainee.

Another exemplary aspect is a control program for causing a computer to perform a method in a rehabilitation support apparatus configured to perform rehabilitation support by executing a training program for urging a trainee to perform a predetermined motion and assisting the trainee to perform the motion, the method including: executing the training program for the trainee; detecting motion data from the motion performed by the trainee; generating training data of the trainee from the motion data; and performing a writing process of writing the training data into an external memory according to a state of the training program.

By executing the program described above, it is possible to perform the training data writing process so as not to interfere with training motions performed by a trainee.

According to the present disclosure, it is possible to provide a rehabilitation support apparatus or the like capable of reducing an operation delay which interferes with training motions performed by a user.

The above and other objects, features and advantages of the present disclosure will become more fully understood from the detailed description given hereinbelow and the accompanying drawings which are given by way of illustration only, and thus are not to be considered as limiting the present disclosure.

### BRIEF DESCRIPTION OF DRAWINGS

Fig. 1 is a perspective view showing a schematic configuration of a walking training apparatus according to a first embodiment;
Fig. 2 shows a functional block diagram of the walking training apparatus according to the first embodiment;
Fig. 3 is a flow chart for a writing process in the walking training apparatus according to the first embodiment;
Fig. 4 is a flow chart showing a specific example of a data writing process;
Fig. 5 is a flow chart showing a specific example of a data writing process;
Fig. 6 is an overall schematic diagram of a walking training system according to a second embodiment;
Fig. 7 is a sequence diagram of the walking training system according to the second embodiment; and
Fig. 8 is a sequence diagram of the walking training system according to the second embodiment.

### DESCRIPTION OF EMBODIMENTS

### <First Embodiment>

A first embodiment according to the present disclosure will be described hereinafter with reference to the drawings.

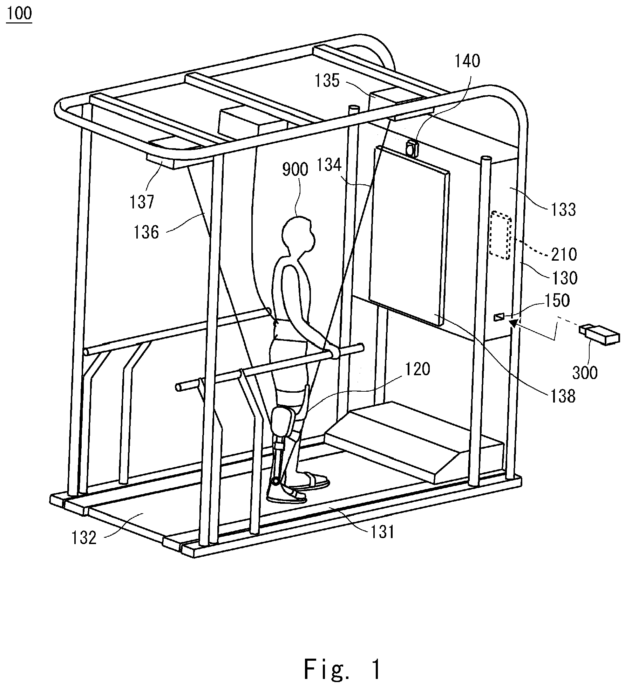

Fig. 1 is a perspective view showing a schematic configuration of a walking training apparatus 100 according to a first embodiment. The walking training apparatus 100 according to this embodiment is, for example, a rehabilitation support apparatus by which a trainee 900 such as a patient having hemiplegia caused by a stroke performs walking training.

The walking training apparatus 100 mainly includes a control panel 133 attached to a frame 130 forming an overall framework, a treadmill 131 on which the trainee 900 walks, and a walking assistance apparatus 120 attached to the diseased leg, i.e., the leg on the paralyzed side of the trainee 900. The frame 130 is disposed on the treadmill 131 mounted on a floor surface in a standing state. The treadmill 131 rotates a ring-shaped belt 132 by using a motor (not shown). The treadmill 131 is an apparatus for urging the trainee 900 to walk. The trainee 900, who performs walking training, gets on the belt 132 and tries to walk according to the movement of the belt 132.

The frame 130 supports the control panel 133 that houses an overall control unit 210 that controls motors or sensors, and a training monitor 138 that is formed by, for example, a liquid-crystal panel, and shows progress of the training etc. to the trainee 900, and so on. Further, the frame 130 supports a front pulling unit 135 in a position located above and in front of the head of the trainee 900 and a rear pulling unit 137 in a position located above and behind the head of the trainee 900.

A camera 140 functions as an image pickup unit for observing the whole body of the trainee 900. The camera 140 is disposed in the vicinity of the training monitor 138 so that it faces the trainee. The camera 140 includes a set of a lens and an image pickup device having such an angle of view that the whole body of the trainee 900 can be captured. The image pickup device is, for example, a CMOS image sensor, and it converts an optical image formed on an image forming surface into an image signal.

One end of a front wire 134 is connected to a winding mechanism of the front pulling unit 135 and the other end of the front wire 134 is connected to the walking assistance apparatus 120. The winding mechanism of the front pulling unit 135 winds up and pays out the front wire 134 according to the movement of the diseased leg by turning on or off the motor (not shown). Similarly, one end of a rear wire 136 is connected to a winding mechanism of the rear pulling unit 137 and the other end of the rear wire 136 is connected to the walking assistance apparatus 120. The winding mechanism of the rear pulling unit 137 winds up and pays out the rear wire 136 by turning the motor (not shown) on/off according to the movement of the diseased leg. By such cooperative operations performed by the front and the rear pulling units 135 and 137, the load (e.g., the weight) of the walking assistance apparatus 120 is cancelled out and hence does not act as a burden on the diseased leg, and a swinging motion of the diseased leg is assisted according to a set level.

For example, an operator who is a training assistant sets an assisting level to a high value for a trainee having severe paralysis. When the assisting level is set high, the front pulling unit 135 winds up the front wire 134 with a relatively strong force in synchronization with the swinging motion of the diseased leg. When the assistance becomes unnecessary as the training proceeds, the operator sets the assisting level to a minimum value. When the assisting level is set to the minimum value, the front pulling unit 135 winds up the front wire 134 with a force by which the weight of the walking assistance apparatus 120 is just cancelled in synchronization with the swinging motion of the diseased leg.

The walking assistance apparatus 120 is attached to the diseased leg of the trainee 900 to assist the trainee 900 in his/her walking by reducing the burden of extending and bending motions in the knee joint of the diseased leg. Specifically, the burden in the knee joint of the diseased leg is reduced by using the motor (not shown) which generates a driving force to assist the extending and bending motions of the knee joint. Further, the walking assistance apparatus 120 includes a sensor and the like for measuring a load on the sole, and outputs their outputs as data on the moving leg to the overall control unit 210.

The control panel 133 includes an external interface 150 for externally inputting and outputting data from and to the walking training apparatus 100. The external interface 150 is, for example, a receptacle connector in conformity with the USB (Universal Serial Bus) standard. For example, an external memory 300 that is an external device is connected with the external interface 150. The external memory is, for example, a USB memory or an external hard disc drive. Note that the specification of the external interface 150 is not limited to the USB standard and other standards can be adopted.

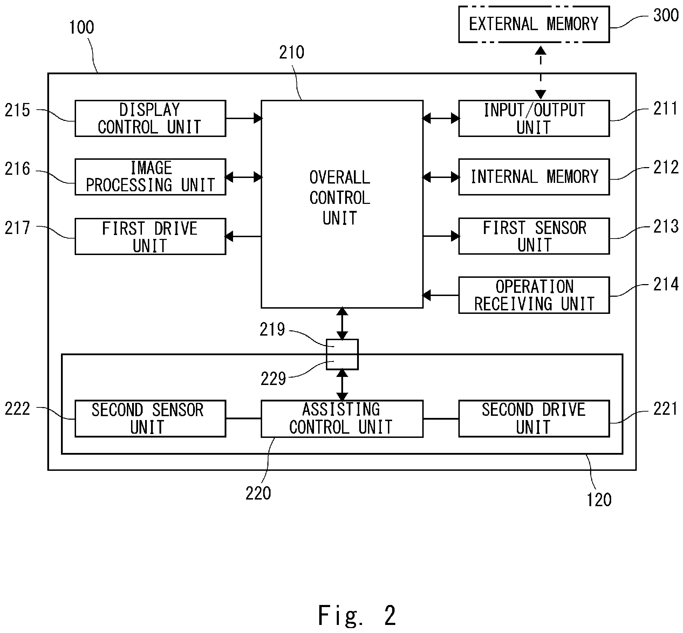

Next, the configuration of the walking training apparatus 100 will be further described with reference to Fig. 2. Fig. 2 is a block diagram showing a configuration of the walking training apparatus 100. The overall control unit 210 is, for example, an MPU, and controls the overall operation of the apparatus by executing a control program loaded from a system memory.

The overall control unit 210 functions as a function performing unit that performs various calculations and controls related to the control. The overall control unit 210 controls the walking training apparatus 100 according to a training program and successively collects information items on the trainee 900. Further, the overall control unit 210 generates training data in a predetermined format from the collected information about the walking training, the state of the apparatus, information attributed to the trainee 900 himself/herself, and so on.

Further, the overall control unit 210 may include a timer. In this case, the overall control unit 210 monitors a lapse of time by the timer when a data writing process is performed. Then, the overall control unit 210 may perform a process for stopping the data writing when the monitored time exceeds a predetermined time. Note that the overall control unit 210 may include a built-in timer or include an external timer connected through a data bus.

An input/output unit 211 includes, for example, a USB interface, and is a communication interface for connecting with an external apparatus. The overall control unit 210 communicates with the external apparatus through the input/output unit 211. By doing so, the overall control unit 210 rewrites a training program, receives commands, and outputs generated training data. For example, the input/output unit 211 connects with an external memory 300 through the external interface 150.

An internal memory 212 stores a training program and cooperates with the overall control unit 210 to perform the training program. Further, the internal memory 212 stores training data which is successively generated by the overall control unit 210 while performing the training program. The internal memory 212 may be composed of, for example, a nonvolatile flash memory, a volatile DRAM (Dynamic Random Access Memory), or a combination thereof.

A first sensor unit 213 includes various sensors disposed in a distributed manner and their drive circuits. The overall control unit 210 performs sensing control by transmitting a sensing start signal and an output request signal for each of sensors to the first sensor unit 213. The overall control unit 210 processes received sensor outputs, records the processed data on the training data, performs feedback for the subsequent control, and so on.

An operation receiving unit 214 receives an input operation performed by the trainee 900 or an operator and transmits an operation signal to the overall control unit 210. The trainee 900 or the operator operates operation buttons provided in the apparatus, a remote controller belonging to the apparatus, or the like, which constitutes the operation receiving unit 214, and thereby provides an instruction to turn on/off a power supply or to start training, enters numerical values for the setting, or selects a menu item. Further, the operation receiving unit 214 may be instructed to perform a training data writing process according to an instruction received from the trainee 900 or the operator.

A display control unit 215 receives a display signal from the overall control unit 210, generates a display image, and displays the generated image on the training monitor 138. The display control unit 215 generates images showing the progress of the training and real-time video images taken by the camera 140 according to the display signal.

An image processing unit 216 generates an image data by image-processing an image signal received from the camera 140 according to an instruction received from the overall control unit 210. Further, the image processing unit 216 can perform a specific image analysis by image-processing the image signal received from the camera 140 according to the overall control unit 210. For example, the image processing unit 216 detects a position of the diseased leg (leg-standing position) in contact with the treadmill 131 by performing the image analysis. Further, the image processing unit 216 can generate image data by performing a trimming or the like in order to adapt image data to the data format of training data. For example, the image processing unit 216 cuts out an area near the foot from the image data when generating moving image data of the moving leg.

A first drive unit 217 includes a motor and its drive circuit for each of the belt 132, the front pulling unit 135, and the rear pulling unit 137, which are disposed in a distributed manner. The overall control unit 210 performs drive control by transmitting drive signals to the respective first drive units 217. For example, the overall control unit 210 adjusts rotating speed of the belt 132 according to the set walking speed and turns on/off the motors of the front and rear pulling units 135 and 137 in synchronization with the timing at which the diseased leg is changed from a leg-standing state to a leg-idling state.

The walking assistance apparatus 120 is attached to the diseased leg of the trainee 900. Further, the walking training apparatus 100 includes a communication connecting IF (Interface) 219 connected to the overall control unit 210 in order to provide an instruction to the walking assistance apparatus 120, receive sensor information therefrom, and so on. Similarly, the walking assistance apparatus 120 is provided with a communication connecting IF 229 which is connected to the communication connecting IF 219 wirelessly or through a wire. The communication connecting IF 229 is connected to an assisting control unit 220 of the walking assistance apparatus 120. The communication connecting IFs 219 and 229 are communication interfaces in conformity with communication standards such as a wireless LAN.

The assisting control unit 220 is, for example, an MPU, and controls the walking assistance apparatus 120 by executing a control program provided from the overall control unit 210. Further, the assisting control unit 220 notifies the overall control unit 210 of a state of the walking assistance apparatus 120 through the communication connecting IFs 219 and 229. Further, the assisting control unit 220, for example, starts/stops the walking assistance apparatus 120 in response to an instruction from the overall control unit 210.

A second drive unit 221 includes a motor of the walking assistance apparatus 120 and its drive circuit. The assisting control unit 220 assists the upper and lower legs of the trainee 900 by sending a drive signal to the second drive unit 221 so that the upper and lower legs open and close relative to each other around a hinge axis. By performing such movements, the assisting control unit 220 assists extending and bending motions of the knee, prevents the knee from accidentally bending, and so on.

A second sensor unit 222 includes various sensors of the walking assistance apparatus 120 and their circuits. Each of the sensors included in the second sensor unit transmits its detected data to the assisting control unit 220. An example of the sensors included in the second sensor unit is a load sensor for detecting magnitude and a distribution of a vertical load that the sole of the trainee 900 receives. Further, another example of the sensors of the second sensor unit is an angle sensor for detecting an angle formed between an upper-leg frame attached to the upper leg of the trainee 900 and a lower-leg frame attached to the lower leg of the trainee 900.

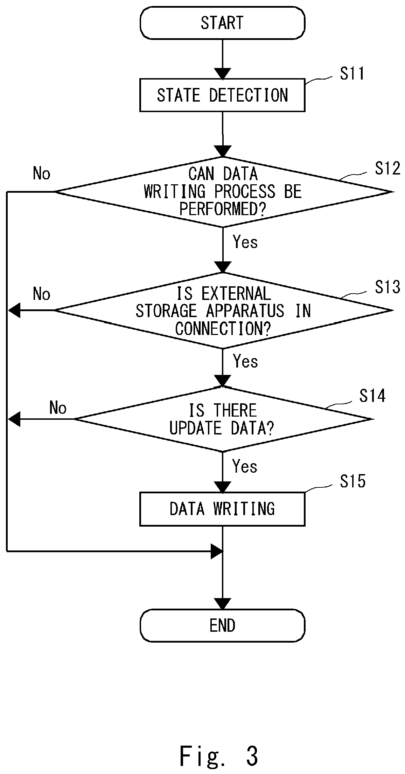

Next, a data writing process performed by the walking training apparatus 100 will be described with reference to Fig. 3. The walking training apparatus 100 writes training data stored in the internal memory 212 into an external memory in response to an instruction from a user or when a predetermined situation occurs. The predetermined situation is, for example, when the power supply of the walking training apparatus 100 is turned on and the walking training apparatus 100 starts, or a predetermined time or the like. When the overall control unit 210 receives an instruction to perform a writing process for writing training data, it performs processes shown in Fig. 3.

First, the overall control unit 210 detects a state of the walking training apparatus 100 (step S11). The state of the walking training apparatus 100 means a state based on predetermined items, for example, an execution state of a training program, a state of each of sensor units, or a state of each of drive units.

Next, the overall control unit 210 makes a determination whether or not a data writing process can be performed according to the detected state of the walking training apparatus 100 (step S12). For example, the overall control unit 210 makes the above determination based on whether or not the training program is being executed in the case where the overall control unit 210 is set to be able to perform the data writing process when it is not executing the training program.

Meanwhile, when the overall control unit 210 is executing the training program and hence the training program process may be delayed by the execution of the data writing process, the overall control unit 210 does not determine that the data writing process can be performed (step S12: No). In this case, the overall control unit 210 terminates the process without performing the data writing.

On the other hand, when the overall control unit 210 determines that the data writing process can be performed (step S12: Yes), the overall control unit 210 determines whether or not the walking training apparatus 100 is connected to the external memory 300 (step S13).

When the overall control unit 210 does not determine that the walking training apparatus 100 is connected to the external memory 300 (step S13: No), the overall control unit 210 terminates the process without performing the data writing.

On the other hand, when the overall control unit 210 determines that the walking training apparatus 100 is connected to the external memory 300 (step S13: Yes), the overall control unit 210 determines whether or not update data is stored in the internal memory 212 (step S14).

When the overall control unit 210 does not determine that the update data is stored in the internal memory 212 (step S14: No), the overall control unit 210 terminates the process without performing the writing data.

On the other hand, when the overall control unit 210 determines that the update data is stored in the internal memory 212 (step S14: Yes), the overall control unit 210 writes the update data, which is stored in the internal memory 212, into the external memory 300 (step S15).

By performing the process described above, it is possible to perform the training data writing process so as not to interfere with the training motions performed by a trainee.

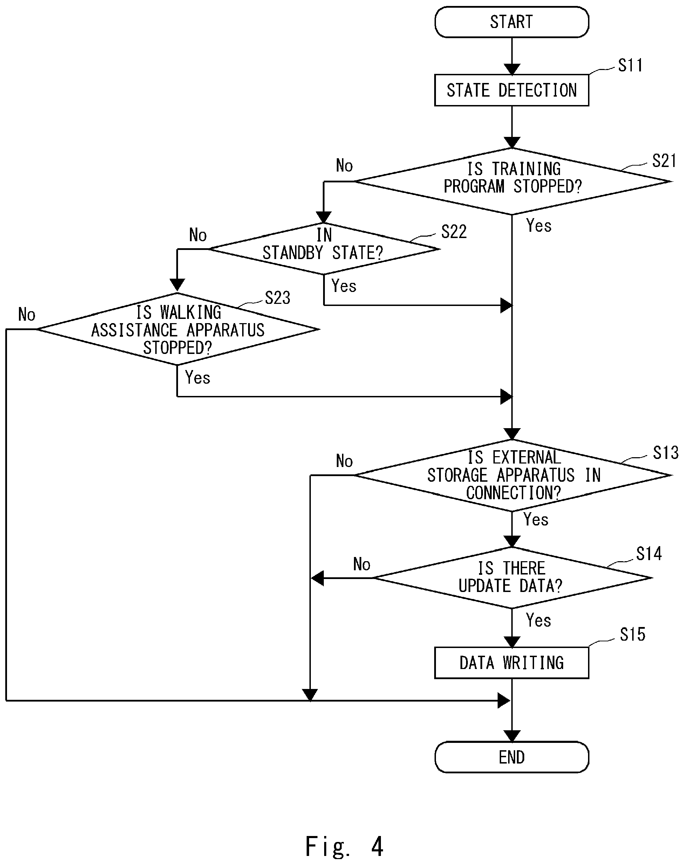

Next, a specific example of the case where it is determined whether or not a data writing process can be performed will be described with reference to Fig. 4. Fig. 4 is a flow chart showing a specific example of a data writing process. Processes performed between steps S11 and S13 in Fig. 4 are different from those in the flowchart shown in Fig. 3.

First, the overall control unit 210 detects a state of the walking training apparatus 100 (step S11). The state of the walking training apparatus 100 means a state based on the following processes, that is, an execution state of a training program and a state of an assistance apparatus.

Next, the overall control unit 210 determines whether or not the training program is stopped based on the detected state of the walking training apparatus 100 (step S21). When the training program is stopped, the trainee 900 is not performing walking training. Thus, the training data writing process performed by the overall control unit 210 does not interfere with the walking training. Therefore, when the overall control unit 210 determines that the training program is stopped (step S21: Yes), the walking training apparatus 100 then determines whether or not the walking training apparatus 100 is connected to the external memory 300 (step S13). Note that processes performed after the step S13 are similar to those described with reference to Fig. 3.

On the other hand, when the overall control unit 210 does not determine that the training program is stopped (step S21: No), the overall control unit 210 then determines whether or not the training program is in a standby mode (step S22). Even after the training program has started, the training data writing process performed by the overall control unit 210 does not interfere with the walking training when the program is not practically executed, such as when the trainee is not practically performing the walking training and hence a screen saver is displayed on the screen. Therefore, when the overall control unit 210 determines that the training program is in a standby state (step S22: Yes), the overall control unit 210 then determines whether or not the walking training apparatus 100 is connected to the external memory 300 (Step S13).

Meanwhile, when the overall control unit 210 does not determine that the training program is in a standby state (step S22: Yes), the overall control unit 210 determines whether or not the walking assistance apparatus 120 is being stopped (step S23). Even in a state that the training program has already started, the training data writing process performed by the overall control unit 210 does not interfere with the walking training when the trainee is not practically performing the walking training and hence the walking assistance apparatus 120 is in a stopped state. Therefore, when the overall control unit 210 determines that the walking assistance apparatus 120 is stopped (step S23: Yes), the overall control unit 210 then determines whether or not the walking training apparatus 100 is connected to the external memory 300 (Step S13).

On the other hand, when the overall control unit 210 does not determine that the walking assistance apparatus 120 is stopped (step S23: No), it is presumed that the training program is being executed and the trainee is performing walking training. Therefore, the overall control unit 210 terminates the process without performing the data writing.

By performing the process described above, it is possible to perform the training data writing process so as not to interfere with training motions performed by a trainee. Note that the process shown in Fig. 4 is an example. That is, among the steps S21 to S23, only one or two of them may be employed. For example, in the case where the step S23 is not employed, when the overall control unit 210 determines that the training program is in a standby state in the step S22, the overall control unit 210 terminates the data writing process. Similarly, when the overall control unit 210 performs the data writing in a state that the training program is being executed or the walking assistance apparatus is being driven, and thereby could interfere with the training motion performed by a trainee, the overall control unit 210 terminates the data writing process.

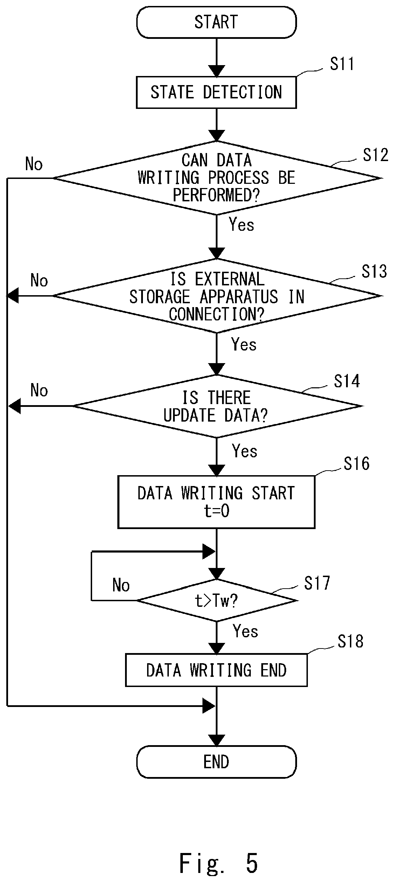

Next, a specific example of a data writing process will be described with reference to Fig. 5. Fig. 5 is a flow chart showing a specific example of a data writing process. Processes performed after step S14 in Fig. 5 are different from those in the flowchart shown in Fig. 3. The processes which are different from those in Fig. 3 will be described below.

In step S14, the overall control unit 210 determines whether or not update data is stored in the internal memory 212 (step S14).

When the overall control unit 210 does not determine that the update data is stored in the internal memory 212 (step S14: No), the overall control unit 210 terminates the process without performing the writing data.

On the other hand, when the overall control unit 210 determines that the update data is stored in the internal memory 212 (step S14: Yes), the overall control unit 210 starts a process of writing the data, which is stored in the internal memory 212, into the external memory 300. In this case, the overall control unit 210 sets a count value of a timer, which the overall control unit 210 monitors, to zero (t=0) (step S16).

Next, the overall control unit 210 continues the data writing process while monitoring the timer (step S17). When the overall control unit 210 does not determine that a time t exceeds a predetermined time Tw (step S17: No), the overall control unit 210 continues the data writing process. On the other hand, when the overall control unit 210 determines that the time t has exceeded the predetermined writing time Tw (step S17: Yes), the overall control unit 210 finishes the data writing process (step S18).

Note that the above predetermined writing time Tw may be changed according to a state of the training program and the like when the overall control unit performs the data writing. For example, there is less possibility that the walking training performed by a trainee is hindered even when the writing time Tw is set long in the case where the training program is stopped, in comparison with the case where the training program is being executed. On the other hand, in the case where the training program is not stopped and is being executed, there is a possibility that the walking training by the trainee might be hindered when the writing time Tw is long. In such a case, the writing time when the training program is stopped may be set shorter than that when the training program is being executed.

By performing the above described process, it is possible to reduce a waiting time for the next process until the data writing process is finished. Thus, in the first embodiment, it is possible to provide a rehabilitation support apparatus capable of reducing an operation delay which interferes with training motions performed by a user.

### <Second Embodiment>

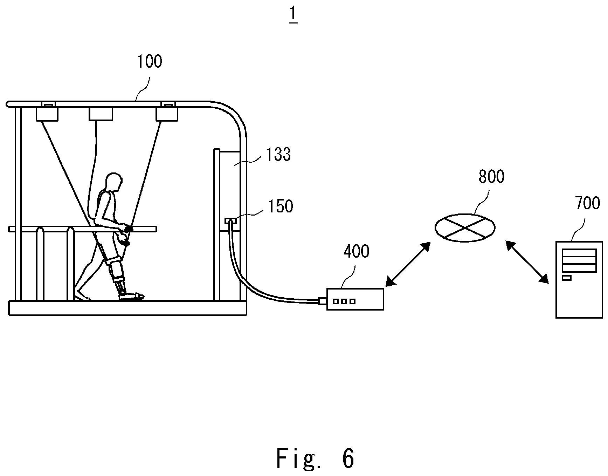

Next, a second embodiment will be described with reference to Fig. 6. Fig. 6 is an overall schematic diagram of a walking training system according to the second embodiment. A walking training system 1 according to the second embodiment further includes an external communication apparatus 400, in addition to the walking training apparatus 100 according to the first embodiment.

The external communication apparatus 400 is an example of medical data communication apparatuses. The external communication apparatus 400 has a function of receiving and storing training data output from the walking training apparatus 100. Further, the external communication apparatus 400 modifies has functions of receiving a modification instruction of training data from server 700 and modifying the training data according to that modification instruction, and a function of transmitting the training data to the server 700. The external communication apparatus 400 is connected to the walking training apparatus 100 by, for example, a USB cable through the external interface 150 so that the external communication apparatus 400 can communicate with the walking training apparatus 100. Further, the external communication apparatus 400 is connected to an Internet network 800 as an example of external networks wirelessly or through a wire so that external communication apparatus 400 can communicate with the Internet network 800.

The server 700 is connected to the Internet network 800 and has a function of accumulating training data received from the external communication apparatus 400 through the Internet network 800. Further, the server 700 has a function of providing accumulated training data to a user in response to an operation of the user. Further, the server 700 may transmit update information and the like of training program to the external communication apparatus 400.

In the above-described walking training system, the walking training apparatus 100 is connected to the Internet network 800 through the external communication apparatus 400 as described above and does not have a communication function of directly connecting to the Internet network 800. That is, the walking training apparatus 100 does not directly output the training data to the external network. By interposing the external communication apparatus 400 between the Internet network 800 and the walking training apparatus 100 as described above, it is possible to prevent training data from being leaked from the walking training apparatus 100. Further, the connection between the walking training apparatus 100 and the external communication apparatus 400 is not limited to connection using a USB cable. That is, various communication standards can be adopted as long as they use communication paths that do not go through any external network.

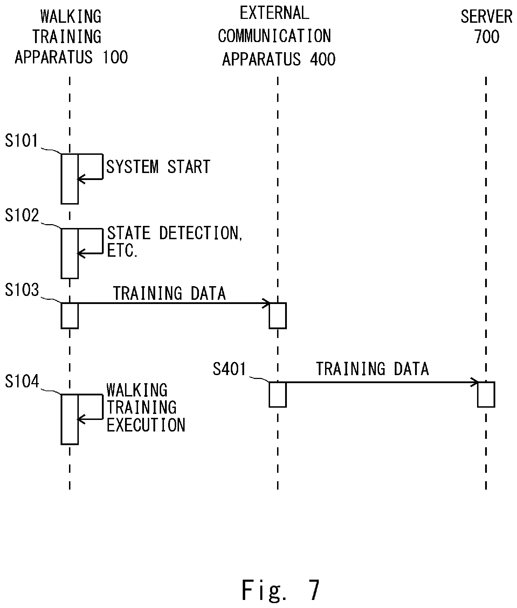

Next, exchanges of data among the apparatuses in the walking training system 1 according to the second embodiment will be described with reference to Fig. 7. Fig. 7 is a sequence diagram of the walking training system according to the second embodiment. In the example in Fig. 7, a data writing process is automatically performed when the walking training apparatus 100 has started the system.

First, in Fig. 7, the walking training apparatus 100 starts the system (step S101). The system may be started in response to an operation of a user or at a predetermined time set by a timer.

Next, the walking training apparatus 100 starts a training data writing process according to a predetermined operation sequence. That is, the overall control unit 210 performs a process such as a process for detecting a state of the walking training apparatus 100 (step S102). The processes performed in this step are, for example, the processes of the steps S11 to S14 in the above-described flowchart shown in Fig. 3 or 4.

Next, the walking training apparatus 100 and the external communication apparatus 400 exchange training data with each other (step S103). Specifically, the walking training apparatus 100 performs a process of writing the training data into the external communication apparatus 400. That is, the walking training apparatus 100 transmits the training data, which is stored in an internal memory, to the external communication apparatus. The external communication apparatus 400 receives the training data from the walking training apparatus 100 and stores the received training data.

When the walking training apparatus 100 and the external communication apparatus 400 complete exchanging training data with each other, the walking training apparatus 100 starts a training program and a trainee performs walking training (step S104). The training program is executed in response to an operation of a trainee, an assistant, etc. Alternatively, the training program may be set to be automatically executed when the training data writing process is completed.

On the other hand, when the walking training apparatus 100 and the external communication complete exchanging the training data, the external communication apparatus 400 and the server 700 exchange the training data with each other (step S401). Specifically, the external communication apparatus 400 transmits the stored training data to the server 700. The server 700 receives the training data from the external communication apparatus 400 and accumulates the received training data.

The process of exchanging training data between the external communication apparatus 400 and the server 700 does not have an effect such as an operation delay on the processing operation of the overall control unit 210 of the walking training apparatus 100. Therefore, the process of exchanging training data between the external communication apparatus 400 and the server 700 may be performed regardless of whether or not walking training is being performed in the walking training apparatus 100. That is, the process of exchanging the training data between the external communication apparatus 400 and the server 700 may be performed while the walking training is being performed in the walking training apparatus 100.

As described above, the walking training system 1 according to the second embodiment can perform a training data writing process so as not to interfere with training motions performed by a trainee, and perform communication with the server 700 so as not to interfere with the training motions performed by the trainee.

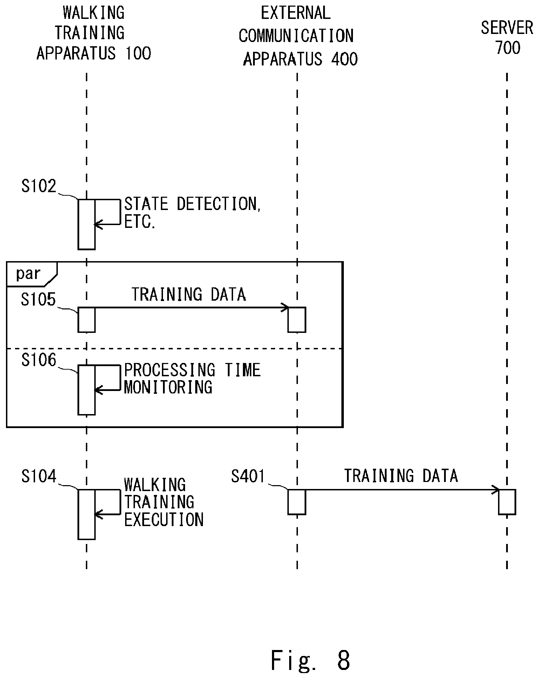

Next, the case where the walking training system 1 according to the second embodiment includes a timer will be described with reference to Fig. 8. Fig. 8 is a sequence diagram of the walking training system according to the second embodiment. Further, in the example shown in Fig. 8, a data writing process is performed at arbitrary timing after the system of the walking training apparatus 100 is started.

First, the walking training apparatus 100 starts a training data writing process, for example, in response to an operation of a user. That is, the overall control unit 210 performs a process such as a process for detecting a state of the walking training apparatus 100 (step S102). The processes performed in this step are, for example, the processes of the steps S11 to S14 in the above-described flowchart shown in Fig. 3 or Fig. 4.

Next, the walking training apparatus 100 and the external communication apparatus 400 exchanges training data with each other (step S105). Then, the overall control unit 210 monitors the processing time while the walking training apparatus 100 and the external communication apparatus 400 exchange the training data with each other (step S106). When the processing time reaches the predetermined time, the overall control unit 210 stops transmitting the training data. Thus, the walking training apparatus 100 and the external communication apparatus 400 stop exchanging the training data with each other.

After the walking training apparatus 100 and the external communication apparatus 400 stop exchanging training data, the external communication apparatus 400 and the server 700 may perform a process of exchanging the training data, which is stored until that time, with each other (step S401). Further, after the walking training apparatus 100 and the external communication apparatus 400 stop exchanging training data with each other, the trainee may perform walking training in the walking training apparatus 100 (step S104).

In the above described structure, the walking training system 1 according to the second embodiment can reduce a waiting time for the next step until the data writing process is finished. Thus, in the second embodiment, it is possible to provide a rehabilitation support apparatus capable of reducing an operation delay which interferes with training motions performed by a user.

Note that the present disclosure is not limited to the above described embodiments and various modifications can be made without departing from the spirit of the present disclosure.

The program can be stored and provided to a computer using any type of non-transitory computer readable media. Non-transitory computer readable media include any type of tangible storage media. Examples of non-transitory computer readable media include magnetic storage media (such as floppy disks, magnetic tapes, hard disk drives, etc.), optical magnetic storage media (e.g. magneto-optical disks), CD-ROM (compact disc read only memory), CD-R (compact disc recordable), CD-R/W (compact disc rewritable), and semiconductor memories (such as mask ROM, PROM (programmable ROM), EPROM (erasable PROM), flash ROM, RAM (random access memory), etc.). The program may be provided to a computer using any type of transitory computer readable media. Examples of transitory computer readable media include electric signals, optical signals, and electromagnetic waves. Transitory computer readable media can provide the program to a computer via a wired communication line (e.g. electric wires, and optical fibers) or a wireless communication line.

From the disclosure thus described, it will be obvious that the embodiments of the disclosure may be varied in many ways. Such variations are not to be regarded as a departure from the spirit and scope of the disclosure, and all such modifications as would be obvious to one skilled in the art are intended for inclusion within the scope of the following claims.

## Claims

1. A rehabilitation support apparatus (100) configured to perform rehabilitation support by executing a training program for urging a trainee to perform a predetermined motion and assisting the trainee to perform the motion, the rehabilitation support apparatus comprising:
a sensor (213, 222) configured to detect motion data from the motion performed by the trainee; and
a control unit (210) configured to execute the training program and generate training data of the trainee from the motion data detected by the sensor, wherein
the control unit performs a writing process of writing the training data into an external memory according to a state of the training program.

2. The rehabilitation support apparatus according to Claim 1, wherein the control unit performs the writing process when it is not executing the training program.

3. The rehabilitation support apparatus according to Claim 1, wherein the control unit performs the writing process when the training program is in a standby state.

4. The rehabilitation support apparatus according to Claim 1, further comprising a motion assistance apparatus configured to be attached to a user's limb and assist the user's training motion, wherein the control unit performs the writing process when the training program is being executed and the motion assistance apparatus is not driven.

5. The rehabilitation support apparatus according to any one of Claims 1 to 4, further comprising a timer configured to measure a time during which the writing process is being performed, wherein the control unit stops the writing process when it detects a predetermined time from the timer.

6. A rehabilitation support system comprising:
the rehabilitation support apparatus according to any one of Claims 1 to 5; and
a communication apparatus configured to transmit the written training data to the external server.

7. A control method performed by a rehabilitation support apparatus configured to perform rehabilitation support by executing a training program for urging a trainee to perform a predetermined motion and assisting the trainee to perform the motion, the control method comprising:
executing the training program for the trainee;
detecting motion data from the motion performed by the trainee;
generating training data of the trainee from the motion data; and
performing a writing process of writing the training data into an external memory according to a state of the training program.

8. The control method according to Claim 7, wherein
the writing process is performed when the training program is not being executed.

9. The control method according to Claim 7, wherein
the writing process is performed when the training program is in a standby state.

10. The control method according to Claim 7, wherein
the rehabilitation support apparatus further comprises a motion assistance apparatus configured to be attached to a user's limb and assist the user's training motion, and
the writing process is performed when the training program is being executing and the motion assistance apparatus is not driven.

11. The control method according to any one of Claims 7 to 10, wherein
the writing process is stopped when a predetermined time has elapsed.

12. A control program for causing a computer to perform a method in a rehabilitation support apparatus configured to perform rehabilitation support by executing a training program for urging a trainee to perform a predetermined motion and assisting the trainee to perform the motion, the method comprising:
executing the training program for the trainee;
detecting motion data from the motion performed by the trainee;
generating training data of the trainee from the motion data; and
performing a writing process of writing the training data into an external memory according to a state of the training program.

13. The control program according to Claim 12, wherein
the writing process is performed when the training program is not being executed.

14. The control program according to Claim 12, wherein
the writing process is performed when the training program is in a standby state.

15. The control program according to Claim 12, wherein
the rehabilitation support apparatus further comprises a motion assistance apparatus configured to be attached to a user's limb and assist the user's training motion, and
the writing process is performed when the training program is being executing and the motion assistance apparatus is not driven.
